# FASCICULE DE BREVET EUROPEEN

(11) **EP 2 640 388 B1**
(45) Date de publication et mention de la délivrance du brevet: **11.10.2017**
(21) Numéro de dépôt: 11794858.8
(22) Date de dépôt: 15.11.2011
(51) Int. Cl.: A61K 31/4704, A61P 19/00, C07D 215/22, C07D 215/56

(54) **DÉRIVÉS DE QUINOLINONE ET UTILISATION POUR TRAITER LES MALADIES CAUSÉES PAR LE DÉRÈGLEMENT DE LA FONCTION DES OSTÉOBLASTES**
QUINOLINONE DERIVATE UND VERWENDUNG ZUR BEHANDLUNG VON ERKRANKUNGEN, DIE DURCH FEHLFUNKTION VON OSTEOBLASTEN HERVORGERUFEN WERDEN
QUINOLINONE DERIVATIVES AND USE TO TREAT CONDITIONS CAUSED BY MALFUNCTION OF OSTEOBLASTS

(30) Priorité: 16.11.2010 FR 1004445
(43) Date de publication de la demande: 25.09.2013
(73) Titulaire: Centre National de la Recherche Scientifique, 75016 Paris (FR)
(72) Inventeur: RUAT, Martial, F-91400 Orsay (FR); FAURE, Hélène, F-91190 Gif-sur-Yvette (FR); GOROJANKINA, Tatiana, F-91190 Gif sur Yvette (FR); MANN, André, F-67540 Ostwald (FR); TADDEI, Maurizio, I-53035 Monteriggioni (IT); MANETTI, Fabrizio, I-53019 Castelnuovo Berardenga (SI) (IT); SOLINAS, Antonio, I-53100 Siena (IT)
(74) Mandataire: Gevers & Orès
(86) Numéro de dépôt international: PCT/IB2011/055096
(87) Numéro de publication internationale: WO 2012/066478

(56) Documents cités:
- WO-A1-00/01386
- UKRAINETS ET AL.: "4-hydroy-2-quinolones 138", CHEMISTRY OF HETEROCYCLIC COMPOUNDS, vol. 43, no. 12, 31 décembre 2007 (2007-12-31), pages 1532-1539, XP002660338,
- LINNAN HE AND P.C. JURS: "Probabilistic neural network multiple classifier system for predicting the genotoxicity of quinolone and quinoline derivatives", CHEM RES TOXICOL, vol. 18, 2 août 2005 (2005-08-02), pages 428-440, XP002660339,
- CHEN J K ET AL: "Small molecule modulation of Smoothened activity", PROC NATL ACAD SCI (US), WASHINGTON, DC; US, vol. 99, no. 22, 29 octobre 2002 (2002-10-29), pages 14071-14076, XP002251705, ISSN: 0027-8424, DOI: 10.1073/PNAS.182542899 cité dans la demande
- DAYAM ET AL.: "Discovery and structure-activity relation ship studies of a unique class of HIV-1 integrase inhibitors", CHEM MED CHEM, vol. 1, 20 décembre 2005 (2005-12-20), pages 238-244, XP002660340,

## Description

La présente invention se rapporte à des composés dérivés de quinolinone capables de moduler l'activité, en particulier d'induire la différenciation, de cellules souches et progénitrices ; ces composés sont utiles pour le traitement de désordres liés à un défaut de différenciation cellulaire ; l'invention se rapporte également à de nouveaux composés parmi ces dérivés de quinolinone et à des compositions pharmaceutiques les contenant.

La réparation de tissus lésés suite à une maladie, un traumatisme ou l'âge utilise de plus en plus des cellules souches ou de progéniteurs qui conservent la capacité de se différencier vers différents types cellulaires. Ces cellules constituent un réservoir capable de renouveler les tissus afin de restaurer les fonctions biologiques. Les cellules souches mésenchymateuses, par exemple, peuvent donner des ostéoblastes, des chondrocytes, des adipocytes, ou des cellules stromales support de l'hématopoïèse.

Les techniques permettant d'orienter ces cellules vers un phénotype choisi sont généralement lourdes (transformation de cellules à l'aide de vecteurs d'expression et nécessité d'exprimer plusieurs gènes) et des solutions alternatives telles que l'utilisation de petites molécules de synthèse inductrices de différenciation constituerait une piste prometteuse.

Parmi les désordres résultant d'un défaut de la différenciation cellulaire figurent ceux liés à un dysfonctionnement de la différenciation des ostéoblastes.

L'os est continuellement renouvelé au cours de la vie par un processus complexe impliquant une résorption par les ostéoclastes et une formation par les ostéoblastes.

Les précurseurs des ostéoblastes sont des cellules pluripotentes également appelées cellules souches mésenchymateuses. Cependant, les mécanismes qui permettent la différenciation de ces cellules vers le lignage ostéoblastique sont complexes et sont d'une grande importance dans la compréhension du développement de l'os. En outre, l'identification de molécules qui induiraient la différenciation des ostéoblastes et stimuleraient leur activité ostéogénique représenterait une piste thérapeutique dans le traitement des maladies de l'os.

En effet, de nombreuses maladies ont pour cause des dérèglements de la fonction ou de la différenciation des ostéoblastes ainsi qu'à des déséquilibres fonctionnels entre les ostéoblastes et les ostéoclastes. La pathologie la plus étudiée -car elle représente un enjeu économique majeur- est l'ostéoporose ; l'ostéoporose est caractérisée par une fragilité excessive du squelette due à une diminution de la masse osseuse et à l'altération de la microarchitecture osseuse. La solidité de l'os résulte d'un équilibre entre l'action de deux types de cellules osseuses : les ostéoblastes qui solidifient l'os et les ostéoclastes (responsables de la résorption osseuse) qui les fragilisent. Une activité dominante des ostéoclastes conduit à l'ostéoporose qui peut résulter soit d'un capital osseux insuffisant en fin de la croissance, soit d'une perte osseuse excessive lors de la vieillesse. La prévention de l'ostéoporose peut se faire via la diminution d'un phénomène physiologique précurseur, l'ostéopénie (baisse de la densité osseuse) qui peut, avant l'ostéoporose peut conduire à des troubles de raréfaction osseuse et à la fragilisation du tissu osseux.

D'autres pathologies sont associées à des dysfonctionnements induisant une perte de masse osseuse, on peut citer :
- l'ostéogenèse imparfaite ; cette maladie est appelée aussi «maladie des os de verre» regroupe des maladies caractérisées par une fragilité osseuse excessive, due à un défaut congénital d'élaboration des fibres collagènes du tissu conjonctif qui forme la trame de l'os. Tous les types se caractérisent par une extrême fragilité des os, signe le plus typique de la maladie ;
- l'hypercalcémie ;
- l'hyperparathyroïdie ;
- l'ostéomalacie ; qui correspond à une décalcification osseuse induite par un défaut de minéralisation (manque d'ions calcium et phosphate) de la trame protéique du squelette ;
- l'ostéonécrose ; qui couvre des affections définies par la mort des cellules du tissu osseux ;
- la maladie osseuse de Paget (ostéite déformante) ; ostéopathie, localisée à un ou plusieurs os, caractérisée par un remodelage osseux excessif aboutissant à une hypertrophie progressive des pièces osseuses et à d'importantes anomalies de la microarchitecture osseuse ;
- l'arthrite rhumatoïde ;
- l'arthrite inflammatoire ;
- l'ostéomyélite ;
- la parodontite ;
- les métastases osseuses.

Le renouvellement du tissu osseux peut également être nécessaire dans des situations où on cherche à accélérer la réparation de l'os comme des fractures, la chirurgie plastique ou la mise en place d'implants notamment dentaires.

La différenciation ostéoblastique est influencée par de multiples voies de signalisation incluant par exemple les voies du TGF-β (transforming growth factor β1), des protéines Hedgehog (Hh), des Wnt, du FGF (fibroblast growth factors), de l'IGF1 (insulin-like growth factor 1) ou des BMPs (bone morphogenetic proteins) (Centrella et al. 1994; Yamaguchi et al. 2000; van der Horst et al. 2003; Fromigue et al. 2004; Hu et al. 2005).

Alors que les BMPs ont été employés avec succès (Johnson and Urist 2000), leur coût est important et les doses nécessaires à la conduite d'une différenciation cellulaire efficace sont bien aux dessus des seuils physiologiques acceptables. Une alternative serait l'utilisation de petites molécules qui permettent de moduler l'activité BMP *in vivo* (Yu et al. 2008).

Le TGF-β a également été décrit comme un acteur majeur de régulation de la balance de l'activité entre ostéoclastes et ostéoblastes. Récemment des inhibiteurs pharmacologiques de son récepteur ont montré une activité stimulante sur les ostéoblastes et inhibitrice sur les ostéoclastes (Mohammad et al. 2009).

Le rôle de l'IGF1 a été bien étudié, mais l'utilisation d'IGF1 humain recombinant, malgré une influence sur le métabolisme osseux présente certains inconvénients. Il ne cible pas spécifiquement le squelette et entraine des effets secondaires qui limitent son utilisation pour les maladies de l'os.

La molécule de signalisation Hedgehog joue un rôle fondamental dans la morphogenèse de nombreux tissus, y compris l'os, ainsi que dans la prolifération cellulaire, et serait impliquée dans le maintien et la réparation tissulaire chez l'adulte (voir les revues de Ingham and McMahon 2001; Wechsler-Reya and Scott 2001; Marti and Bovolenta 2002; Lum and Beachy 2004; Varjosalo and Taipale 2008).

La stimulation de la voie Hedgehog permet l'induction de l'ostéogenèse dans différents modèles. Plusieurs molécules agonistes ont été étudiées :
- les protéines Hedgehog et des polypeptides dérivés qui stimulent la différenciation ostéoblastique en agissant sur la protéine Patched (Spinella-Jaegle et al. 2001; Guan et al. 2009) ;
- la purmorphamine qui permet d'activer des ostéoblastes humains en culture (Wu et al. 2004; Beloti et al. 2005) ;
- des molécules organiques de petite taille comme le SAG (Chen et al. 2002) ;
- les molécules Hh Agl.2 (Frank-Kamenetsky et al. 2002) ;
- des dérivés des oxystérols (Corcoran and Scott 2006; Amantea et al. 2008) qui induisent la différenciation ostéoblastique et la formation de l'os (Aghaloo et al. 2007; Dwyer et al. 2007; Yu et al. 2008).

Par ailleurs, la Demande Internationale WO 00/01386 décrit des inhibiteurs de la Farnesyl Protein Transferase qui présentent un intérêt pour le développement de médicament contre les arthropathies.

Il demeure toutefois utile d'identifier de nouvelles molécules permettant de moduler la différenciation cellulaire, notamment des molécules ayant une activité ostéogénique, et permettant d'allier une bonne activité et un coût limité ; de telles molécules présenteraient un intérêt particulier pour traiter les pathologies liées à l'os.

Les Inventeurs ont identifiés des composés de formule générale (I) capables d'induire une différenciation cellulaire, en particulier ostéoblastique ; ces composés représentent donc de nouveaux agents stimulant la différenciation de cellules souches ou de cellules progénitrices vers les ostéoblastes ou d'autres types cellulaires.

On entend par cellule souche une cellule indifférenciée, embryonnaire ou adulte, capable de donner des cellules spécialisées par différenciation cellulaire et pouvant se renouveler pratiquement indéfiniment. On utilisera préférentiellement des cellules souches adultes.

Les cellules progénitrices sont des cellules adultes pluripotentes, c'est-à-dire dont la différenciation peut conduire à plusieurs types cellulaires.

Dans la présente demande, on considère que des cellules sont engagées dans une différenciation ostéoblastique -elles sont alors qualifiées de "cellules à phénotype ostéoblastique"-, lorsqu'elles produisent la phosphatase alcaline. De telles cellules sont suffisamment engagées dans la différenciation ostéoblastique pour que la poursuite de leur incubation dans le milieu nutritif et/ou leur implantation permette à au moins une partie desdites cellules d'avancer dans la différenciation, jusqu'à la différenciation terminale (avec production d'une matrice extracellulaire minéralisée). La mise en évidence des propriétés caractéristiques de ces cellules (à savoir production de phosphatase alcaline) peut être effectuée de façon classique, par exemple à l'aide des tests mentionnés dans la partie expérimentale ci-après.

En conséquence, la présente invention a pour objet les composés de formule générale (I) suivante : 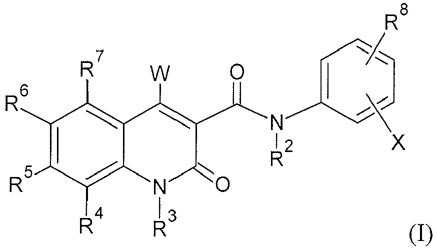 dans laquelle :
- X, positionné en ortho, méta ou para, représente -H, -OH, -NH₂, un atome d'halogène, de préférence, le chlore ou le brome, un radical alkyle consistant en une chaine carbonée de 1 à 10 atomes de carbones, linéaire ou ramifiée, un radical alcoxy (de formule -O-alkyle où le groupe alkyle est tel que défini précédemment), un cycloalkyle de 3 à 8 atomes de carbone ou un groupement aryle
- R⁸, positionné en ortho, méta ou para, sur un carbone différent de celui qui porte le radical X, représente : -(C=O)-NH-R¹, -(C=O)-O-R¹ ou -NH-(C=O)-R¹ ;
- W représente -H, -OH, -NH₂ ou un atome d'halogène, de préférence, le chlore ou le brome ;
- R¹, R² et R³ identiques ou différents et indépendamment les uns des autres, représentent :
   - un atome d'hydrogène ; ou
   - un groupe alkyle consistant en une chaine carbonée de 1 à 10 atomes de carbones, linéaire ou ramifiée, éventuellement insaturée par une ou plusieurs double ou triple liaisons, éventuellement substituée par un ou plusieurs hétéroatomes tels que O et S, par un ou plusieurs atomes d'halogènes ou par un ou plusieurs groupements aryles ou hétéroaryles, de préférence, un groupement pyridine ; ou
   - un cycloalkyle de 3 à 8 atomes de carbones éventuellement substitué par un radical alkyle consistant en une chaine carbonée de 1 à 10 atomes de carbone, linéaire ou ramifiée ou par un radical alcoxy (de formule -O-alkyle où le groupe alkyle est tel que défini précédemment) ;
- R⁴, R⁵, R⁶ et R⁷ identiques ou différents et indépendamment les uns des autres, sont choisis parmi -H, -Cl, -Br, -I, -CN, -NO₂, un radical alkyle consistant en une chaine carbonée de 1 à 10 atomes de carbone, linéaire ou ramifiée, un radical alcoxy (de formule -O-alkyle où le groupe alkyle est tel que défini précédemment) ou un cycloalkyle de 3 à 8 atomes de carbone, par exemple un cyclopropyle, un cyclopentyle, un cyclohexyle ;
étant entendu que R³ et R⁴ peuvent être fusionnés pour former, avec les atomes de carbone et d'azote adjacent du cycle quinoline qui les porte, un cycle à 5 ou 6 chaînons ;
pour leur utilisation pour induire la différenciation cellulaire de cellules souches ou progénitrices et plus particulièrement pour leur utilisation pour le traitement des maladies causées par le dérèglement de la fonction ou de la différenciation des ostéoblastes et/ou par le déséquilibre fonctionnel entre les ostéoblastes et les ostéoclastes, ladite utilisation permet d'induire la différenciation cellulaire de cellules souches ou progénitrices.

Au sens de la présente invention, on entend par alkyle un groupement aliphatique hydrocarboné saturé, linéaire ou ramifié, ayant de 1 à 10 atomes de carbone, de préférence de 3 à 8 atomes de carbone.

Le terme "ramifié" signifie qu'au moins un groupe alkyle inférieur de 1 à 6 atomes de carbones, tel qu'un méthyle ou un éthyle, est porté par une chaîne alkyle linéaire.

Par atome d'halogène, on entend un atome de brome, de chlore, d'iode ou de fluore ; les désignations brome et chlore étant préférées.

Par groupement aryle, on entend tout groupe fonctionnel ou substituant dérivé d'au moins un cycle aromatique ; on peut mentionner les groupes phényle, benzylcyclobutène, pentalène, naphthalène, benzylphényle, et anthracène.

Par groupement hétéroaryle, on entend tout groupe fonctionnel ou substituant dérivé d'au moins un cycle aromatique tel que défini ci-dessus et contenant au moins un hétéroatome choisi parmi P, S, O et N ; parmi les groupes hétéroaryles, on peut mentionner les groupes furane, pyridine, pyrrole, thiophène, imidazole, pyrazole, oxazole, isoxazole, thiazole, pyridine, pyrazine, pyrimidine, pyridazine, benzofurane, isobenzofurane, indole, isoindole, benzothiophène, benzo[c]thiophène, benzimidazole, indazole, benzoxazole, benzisoxazole, benzothiazole, quinoléine, isoquinoléine, quinoxaline, quinazoline, cinnoline, purine, et acridine.

De préférence, les composés de formule générale (I) sont tels que le radical R³ est un radical alkyle de 3 à 8 atomes de carbones tel que le radical hexyle ; le radical R² est un atome d'hydrogène ou un radical alkyle de 1 à 3 atomes de carbone ; que les radicaux R⁴, R⁵, R⁶ et R⁷ représentent un atome d'hydrogène ou un radical alkyle de 1 à 3 atomes de carbone.

A titre de composés de formulé générale (I), on peut en particulier citer de façon non limitative :
- le propyl 4-(1-hexyl-4-hydroxy-2-oxo-1,2-dihydroquinoline-3-carboxamido)-benzoate (**composé 1**) : 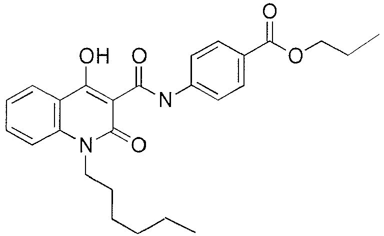
- l'éthyle 4-(1-benzyle-4-hydroxy-2-oxo-1,2-dihydroqumoline-3-carboxamido)-benzoate (**composé 2**) : 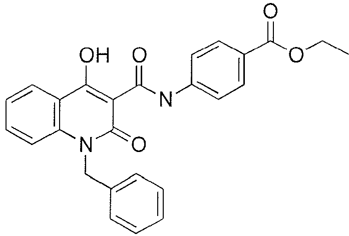
- l'éthyle 4-(1-hexyl-4-hydroxy-2-oxo-1,2-dihydroquinoline-3-carboxamido)-benzoate (**composé 3**) : 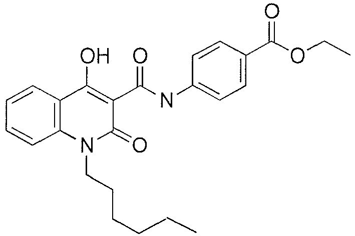
- l'acide 4-(1-hexyl-4-hydroxy-2-oxo-1,2-dihydroquinoline-3-carboxamido)-benzoique (**composé 4**) : 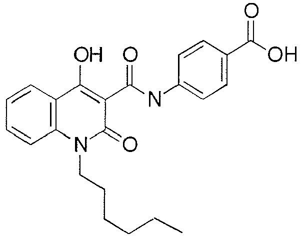
- le butyl 4-(1-hexyl-4-hydroxy-2-oxo-1,2-dihydroquinoline-3-carboxamido)-benzoate (**composé 5**) : 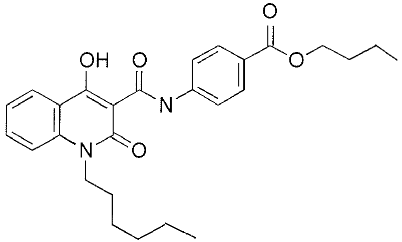
- le tert-butyl 4-(1-hexyl-4-hydroxy-2-oxo-1,2-dihydroquinoline-3-carboxamido)-benzoate (**composé 6**) : 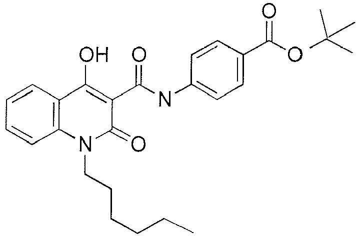
- le benzyle 4-(1-hexyl-4-hydroxy-2-oxo-1,2-dihydroquinoline-3-carboxamido)-benzoate (**composé 7**) : 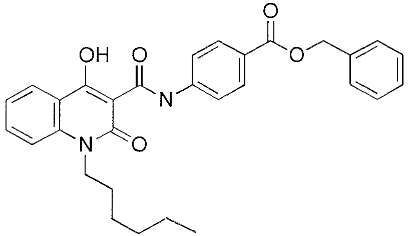
- le N-(4-(butylcarbamoyl)phényl)-1-hexyl-4-hydroxy-2-oxo-1,2-dihydroquinoline-3-carboxamide (**composé 8**) : 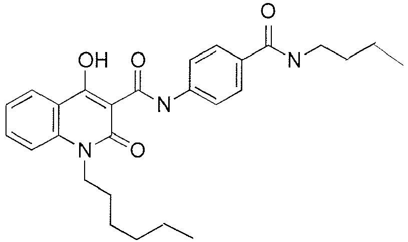
- l'isopropyl-4-(1-hexyl-4-hydroxy-2-oxo-1,2-dihydroquinoline-3-carboxamido)-benzoate (**composé 9**) :

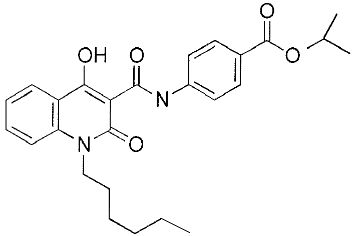

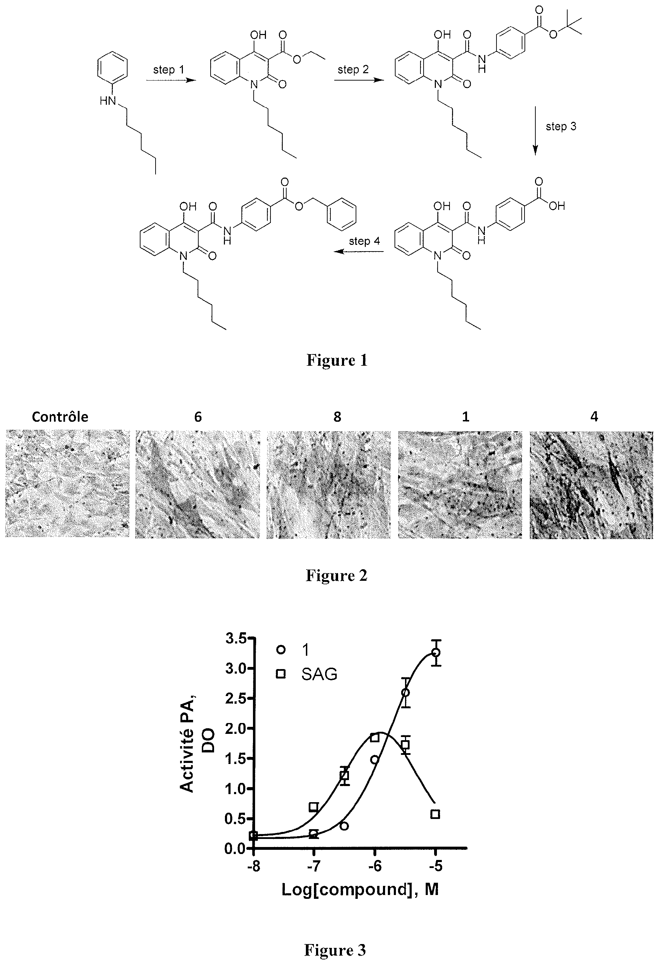

Les composés de formule générale (I) conformes à l'invention peuvent être préparés selon le schéma de synthèse représenté à la **Figure 1****.**

Conformément au schéma de synthèse de la **Figure 1**, dans une première étape, une aniline est condensée avec le triethyl methane tricarboxylate sous irradiation micro-ondes pour obtenir des quinolinones carboxylates ; ensuite, les quinolones sont condensées avec la 4-*tert*-butyl-aniline sous irradiation micro-ondes pour donner des esters carboxamido-quinolinones ; l'étape suivante consiste à saponifier la fonction ester de ces composés pour conduire à un acide point de départ pour différentes modifications telles qu'illustré dans les exemples expérimentaux.

Les composés de formule générale (I) selon l'invention possèdent la propriété d'induire la différenciation de cellules mésenchymateuses en ostéoblastes.

A ce titre, ces composés présentent un intérêt pour la préparation d'implants obtenus à partir de cellules autologues dont on aura induit la différenciation en ostéoblates ; ces implants sont utiles pour remédier aux pertes de tissu osseux consécutives à des blessures et/ou à des opérations chirurgicales.

Les composés de formule générale (I) présentent donc un intérêt particulier pour le traitement des maladies causées par le dérèglement de la fonction ou de la différenciation des ostéoblastes et/ou par le déséquilibre fonctionnel entre les ostéoblastes et les ostéoclastes.

Plus particulièrement, ces composés sont utiles pour le traitement de l'ostéoporose ; et des désordres tels que la fragilité du squelette et/ou raréfaction osseuse et/ou la fragilisation du tissu osseux résultant de l'ostéopénie ; l'ostéogenèse imparfaite ; l'hypercalcémie ; l'hyperparathyroïdie ; l'ostéomalacie ; l'ostéonécrose ; la maladie osseuse de Paget (ostéite déformante) ; l'arthrite rhumatoïde ; l'arthrite inflammatoire ; l'ostéomyélite ; la parodontite ; les métastases osseuses.

En outre, les molécules agissant de façon plus générale sur la différenciation de cellules progénitrices sont utiles dans le traitement médical ou chirurgical (chirurgie plastique ou réparatrice, greffe de tissus ou d'organes) de nombreuses pathologies aiguës, subaiguës ou chroniques, génétiques ou acquises -impliquant un dysfonctionnement tissulaire- pour induire la formation, la régénération, la réparation et/ou l'augmentation de l'activité de tissus tels que de manière non-limitative : le tissu nerveux [système nerveux central (cerveau) et périphérique (neurones sensoriels, moteurs, sympathiques], l'os, le cartilage, les testicules, le foie, la rate, l'intestin, le pancréas, les reins, les muscles lisses et squelettiques, le coeur, les poumons, la peau et le système pileux, les muqueuses, les cellules sanguines et les cellules du système immunitaire. A titre d'exemple non-limitatif de ces pathologies, on peut citer notamment les neuropathies et les maladies neuromusculaires associées, le diabète, l'alopécie, les brûlures, les ulcérations (peau et muqueuse) et les troubles de la spermatogenèse.

La présente invention se rapporte également à un procédé de préparation de cellules ostéoblastiques à partir de cellules souches ou de cellules progénitrices, telles que des cellules mésenchymateuses, comprenant l'étape consistant à faire incuber pendant un temps suffisant lesdites cellules dans un milieu nutritif liquide permettant le développement desdites cellules, ledit milieu nutritif contenant en solution au moins un composé de formule générale (I).

En pratique, on fait incuber lesdites cellules dans le milieu de culture, dans des conditions standard permettant leur développement, c'est-à-dire non seulement leur survie mais aussi leur prolifération et/ou leur différenciation. Les conditions standards de culture de cellules humaines sont connues : par exemple température de 37°C environ ; atmosphère air-COss 95 : 5 ; pH voisin de la neutralité.

Le milieu de culture utilisé est un milieu nutritif liquide classique contenant les ingrédients nécessaires au développement de cellules de mammifères. Ces ingrédients sont connus. Ce sont principalement des sels minéraux (notamment Na, K, Mg, Ca et éventuellement Cu, Fe, Zn), des acides aminés, des vitamines et des sources de carbone (par exemple glucose). On peut utiliser notamment un milieu nutritif tel que le milieu minimum essentiel MEM de EAGLE, complémenté avec du sérum de veau foetal ou, de préférence, avec du sérum humain autologue.

On peut également utiliser des milieux nutritifs plus élaborés, du type DME (milieu de EAGLE modifié par DULBECCO), éventuellement en mélange avec le milieu F 12 de HAM, avec ou sans sérum, et de préférence en présence de sérum autologue.

Ces milieux de culture peuvent être additionnés de facteurs ostéoinducteurs, comme les facteurs BMP, mais, comme indiqué ci-dessus, ces facteurs ne sont pas nécessaires pour induire la différenciation ostéoblastique des cellules humaines utilisées dans le procédé de l'invention. On peut donc utiliser des milieux exempts de facteurs ostéoinducteurs.

De préférence, les milieux de culture utilisés sont additionnés de facteurs ostéopromoteurs tels que l'acide ascorbique et les bêta-glycérophosphates (par exemple de sodium ou de calcium).

La présente invention se rapporte en outre aux nouveaux composés de formule générale (Ia) : 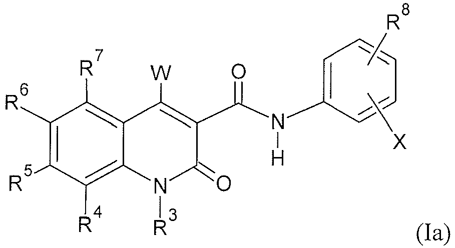 dans laquelle :
- X, positionné en ortho, méta ou para, représente -H, -OH, -NH₂, un atome d'halogène de préférence le chlore ou le brome, un radical alkyle consistant en une chaine carbonée de 1 à 10 atomes de carbones, linéaire ou ramifiée, un radical alcoxy (de formule -O-alkyle où le groupe alkyle est tel que défini précédemment), un cycloalkyle de 3 à 8 atomes de carbone ou un groupement aryle ;
- R⁸, positionné en ortho, méta ou para, sur un carbone différent de celui qui porte le radical X, représente : -(C=O)-NH-R¹, -(C=O)-O-R¹ ou -NH-(C=O)-R¹ ;
- W représente -H, -OH, -NH₂ ou un atome d'halogène, de préférence, le chlore ou le brome ;
- R¹ et R³, identiques ou différents, et indépendamment l'un de l'autre, représentent un groupe alkyle consistant en une chaine carbonée de 3 à 10 atomes de carbone, linéaire ou ramifiée, éventuellement insaturée par une ou plusieurs double ou triple liaisons, éventuellement substituée par un ou plusieurs hétéroatomes tels que O et S, par un ou plusieurs atomes d'halogènes ou par un ou plusieurs groupements aryles ou hétéroaryles, de préférence, un groupement pyridine ;
- R⁴, R⁵, R⁶ et R⁷ identiques ou différents et indépendamment les uns des autres, sont choisis parmi -H, -Cl, -Br, -I, -CN, -NO₂, un radical alkyle consistant en une chaine carbonée de 1 à 10 atomes de carbone, linéaire ou ramifiée, un radical alcoxy (de formule -O-alkyle où le groupe alkyle est tel que défini précédemment) ou un cycloalkyle de 3 à 8 atomes de carbone, par exemple un cyclopropyle, un cyclopentyle, un cyclohexyle ;
étant entendu que R³ et R⁴ peuvent être fusionnés pour former, avec les atomes de carbone et d'azote adjacent du cycle quinoline qui les porte, un cycle à 6 chaînons ;
à l'exclusion du butyl 4-(1-propyl-4-hydroxy-2-oxo-1,2-dihydroquinoline-3-carboxamido)-benzoate (décrit dans Linnan et al., 18, Chem. Res. Toxicol, 2005, 428-440) en tant que tels et pour leur utilisation en tant que médicaments.

Les radicaux R⁵ et R⁷ préférés sont choisis, indépendamment l'un de l'autre, parmi -H, -Cl, -Br, -CN, un radical alkyle ou un radical alcoxy.

La posologie utile variera en fonction de l'affection à traiter, de la voie et du rythme d'administration, ainsi que de la nature et du poids du sujet à traiter (humain ou animal).

La présente invention se rapporte en outre à des compositions pharmaceutiques caractérisées par le fait qu'elles comprennent, à titre de principe actif, au moins un composé de formule générale (Ia) selon l'invention, et au moins un excipient pharmaceutiquement acceptable.

Au sein des compositions pharmaceutiques conformes à l'invention, le ou les composés de formule générale (Ia) sont de préférence utilisés en une quantité permettant d'administrer des doses unitaires comprises entre 1 mg et 2 g environ.

L'homme du métier choisira un ou plusieurs excipients pharmaceutiquement acceptables en fonction de la voie d'administration de la composition pharmaceutique. Bien entendu, l'homme de l'art veillera, à cette occasion, à ce que le ou les excipients utilisés soient compatibles avec les propriétés intrinsèques attachées à la composition conforme à la présente invention.

En outre, la forme du médicament ou de la composition pharmaceutique (par exemple, une solution, une suspension, une émulsion, des comprimés, des gélules, des suppositoires, etc...) dépendra de la voie d'administration choisie.

Ainsi, au sens de la présente invention, le médicament ou la composition pharmaceutique peut être administré par n'importe quelle voie appropriée, par exemple par la voie orale, anale, locale, systémique, intraveineuse, intramusculaire ou mucosale, ou bien en utilisant un patch, ou encore sous forme encapsulée dans, ou immobilisée sur, des liposomes, des microparticules, des microcapsules, et analogues.

On peut notamment citer, à titre d'exemples non limitatifs d'excipients appropriés pour une administration par voie orale, le talc, le lactose, l'amidon et ses dérivés, la cellulose et ses dérivés, les polyéthylèneglycols, les polymères d'acide acrylique, la gélatine, le stéarate de magnésium, des matières grasses animales, végétales ou synthétiques, les dérivés de la paraffine, les glycols, les stabilisants, les conservateurs, les anti-oxydants, les agents mouillants, les anti-agglomérants, les dispersants, les émulsionnants, les agents modifiants du goût, les agents de pénétrations, de solubilisation, etc...

Les techniques de formulation et d'administration des médicaments et compositions pharmaceutiques sont bien connues dans la technique ici considérée, l'homme du métier pouvant notamment se référer à l'ouvrage Remington's Pharmaceutical Sciences, (21st edition).

Outre les dispositions qui précèdent, l'invention comprend encore d'autres dispositions qui ressortiront de la description qui va suivre, qui se réfère à des exemples de synthèse des composés de formule générale (I), à un exemple de mise en oeuvre des composés de formule générale (I) selon la présente invention, ainsi qu'aux dessins annexés dans lesquels :
La **Figure 1** représente le schéma de synthèse des composés de formule générale (I).
La **Figure 2** illustre la détection histochimique de la phosphatase alcaline induite suite à la différenciation des cellules souches mésenchymateuses sous l'action des composés de formule générale (I). Les cellules ont été cultivées en présence de 10 µM des composés 1, 4, 6 ou 8 indiqué ou du solvant (contrôle) pendant 6 jours dans le milieu de culture habituel. Les cellules ont ensuite été colorées par histochimie pour révéler la présence de la phosphatase alcaline (marquage rouge apparaissant sous forme de taches plus foncées) marqueur de l'ostéogenèse puis photographiées.
La **Figure 3** illustre l'effet du composé 1 sur l'activité enzymatique de la phosphatase alcaline indicateur de l'effet de ce composé sur la différenciation des cellules souches mésenchymateuses C3H10T1/2. La différenciation induite par le composé 1 est mesurée par l'activité enzymatique de la phosphatase alcaline (DO à 415 nm) (ronds). A titre de comparaison, la différenciation des cellules obtenue dans la même expérience avec le SAG est présentée (carrés). Une courbe représentative ± SD sur 5 expériences indépendantes est présentée.
   Les courbes obtenue pour les composé de 2 à 9 sont présentées sur les Figures 4 à 11. Tous ces composés permettent la stimulation de la différenciation avec des différences caractéristiques pour chaque composé. Le maximum d'activité est observé à 10 µM pour la plupart d'entre eux.
**Figure 4** **:** Différenciation des cellules C3H10T1/2 par le composé 2. La réponse au composé 2 a été évaluée dans les mêmes conditions expérimentales que celles mises en oeuvre pour le composé 1. A titre de comparaison, la différenciation des cellules obtenue dans la même expérience avec le composé 1 (10 µM) est indiquée par un losange. Une courbe représentative sur 2-3 expériences indépendantes.
   L'effet des composés suivants a également été testé dans les mêmes conditions expérimentales que celles mises en oeuvre pour le composé 1 :
**Figure 5** **:** Différenciation des cellules C3H10T1/2 induite par le composé 3.
**Figure 6** **:** Différenciation des cellules C3H10T1/2 induite par le composé 4.
**Figure 7** **:** Différenciation des cellules C3H10T1/2 induite par le composé 5.
**Figure 8** **:** Différenciation des cellules C3H10T1/2 induite par le composé 6.
**Figure 9** **:** Différenciation des cellules C3H10T1/2 induite par le composé 7.
**Figure 10** : Différenciation des cellules C3H10T1/2 induite par le composé 8.
**Figure 11** **:** Différenciation des cellules C3H10T1/2 induite par le composé 9.

### EXEMPLE 1 : SYNTHÈSES DE DIFFÉRENTS COMPOSÉS DE FORMULE (I)

### Préparation de l'éthyl 1-hexyl-4-hydroxy-2-oxo-1,2-dihydroquinoline-3-carboxylate

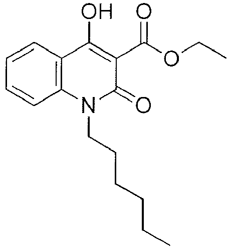

Dans un ballon équipé d'un condenseur, du N-hexylaniline (355 mg, 2 mmol) est ajouté à une solution d'acide triéthylester methanetricarboxylique (1,35 ml, 6,4 mmol). Le mélange réactionnel résultant est placé dans un four à micro-ondes CEM Discovery et irradié dans le ballon ouvert puis l'éthanol formé est distillé (les paramètres sont les suivants : puissance = 250 W, température = 225°C, temps d'exécution = 5 min, temps de maintien = 15 min).

Après chauffage aux micro-ondes, le brut réactionnel est purifié sur colonne chromatographique, avec de l'éther de pétrole : acétate d'éthyle à 4:1. Le produit cristallin obtenu est séché pour donner le composé : rendement = 430 mg (69%). 1H NMR (CDCl3): ∂ 8.16 (d, J=8Hz, 1H), 7.64 (m, 1H), 7.27-7.19 (m, 2H), 4.48 (q, J= 8Hz, 2H), 4.18 (t, J=8Hz, 2H), 1.72-0.86 (m, 14H).

### I.A. tert-Butyl 4-(1-hexyl-4-hydroxy-2-oxo-1,2-dihydroquinoline-3-carboxamido)-benzoate (composé 6)

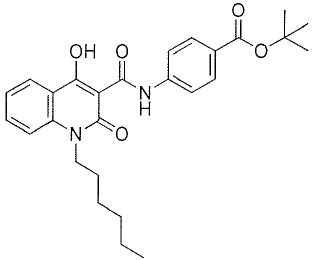

Du 1-hexyl-4-hydroxy-2-oxo-1,2-dihydroquinoléine-3-carboxylate d'éthyle (159 mg, 0,5 mmol) et du tert-butyle 4-aminobenzoate (193 mg, 1 mmol) sont mis en suspension dans du toluène anhydre (4 ml) et sont chauffés au micro-onde récipient ouvert (les paramètres sont les suivants : puissance = 200 W, la température = 120°C, temps d'exécution = 7 min, temps de maintien = 10 min).

A la fin de la réaction, 2 / 3 du solvant sont évaporés sans condensateur. Le brut réactionnel est purifié par colonne chromatographique, utilisant de l'éther de pétrole 4:1: acétate d'éthyle. Le produit cristallin obtenu est séché pour donner le composé du titre: rendement = 195 mg (84%). 1H NMR (DMSO): ∂ 8.16 (d, J=8Hz, 1H), 7.90-7.75 (m, 6H), 7.38 (m, 1H), 4.25 (bs, 2H), 1.60 (bs, 2H), 1.52 (s, 9H), 1.39 (bs, 2H), 1.28 (bs, 5H), 0.84 (bs, 2H).

### I.B. Acide 4-(1-Hexyl-4-hydroxy-2-oxo-1,2-dihydroquinoline-3-carboxamido)-benzoique (composé 4)

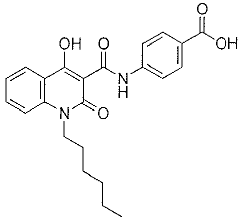

A une solution d'acide trifluoroacétique (TFA) refroidi à 0°C, est ajouté du tert-butyle 4-(1-hexyl-4-hydroxy-2-oxo-1,2-dihydroquinoléine-3-carboxamido) benzoate (83 mg, 0,18 mmol) de façon fractionnée ; le mélange est laissé à réagir pendant 3 heures. Le produit brut obtenu est lavé avec de l'éther éthylique jusqu'à la formation d'un solide cristallin blanc. Le produit est séché pour donner un rendement de 61 mg (83%). 1H NMR (DMSO): ∂ 8.16 (d, J=8Hz, 1H), 7.96-7.65 (m, 6H), 7.38 (m, 1H), 4.27 (bs, 2H), 1.59 (bs, 2H), 1.39 (bs, 2H), 1.28 (bs, 5H), 0.84 (bs, 2H).

### I.C. Benzyle 4-(1-hexyl-4-hydroxy-2-oxo-1,2-dihvdroquinoline-3-carboxamido)-benzoate (composé 7)

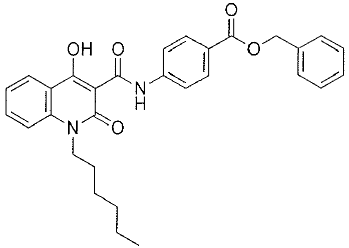

Une solution d'acide 4-(1-hexyl-4-hydroxy-2-oxo-1,2-dihydroquinoléine-3-carboxamido) benzoïque (41 mg, 0,1 mmol) dans du THF est refroidie à 0°C, et de l'alcool benzylique (21 mg, 20 µl, 0,2 mmol) est ajouté, ainsi que de l'EDCI (19 mg, 0,1 mmol) et de la diméthylamino pyridine (3 mg, 0,02 mmol). Le mélange réactionnel est laissé sous agitation pendant une nuit. La phase organique est lavée avec de l'eau et évaporée sous pression réduite. Le produit brut est purifié par chromatographie sur colonne, en utilisant l'éther de pétrole 4:1: acétate d'éthyle, avec un rendement de 30 mg (60%). 1H NMR (DMSO): ∂ 8.16 (d, J=8Hz, 1H), 8.04 (d, J=8Hz, 2H), 7.83 (d, J=8Hz, 3H), 7.68 (d, J=8Hz, 1H), 7.46-7.25 (m, 6H), 7.38 (m, 1H), 5.30 (s, 2H), 4.28 (bs, 2H), 1.59 (bs, 2H), 1.41-1.15 (m, 6H), 0.84 (bs, 3H).

### EXEMPLE II - MISE EN ÉVIDENCE DE L'EFFET DES COMPOSÉS DE FORMULE GENERALE (I) SUR L'OSTÉOGENÈSE

L'effet des composés de formule générale (I) conformes à l'invention sur la stimulation de l'ostéogenèse a été déterminé *in vitro* par analyse de la différenciation de la lignée de cellules mésenchymateuses pluripotentes C3H10T1/2.

### II.1) Matériels et méthodes

### Dissolution des composés, culture cellulaire.

Les composés de formule (I) à tester ont été dissous dans le diméthylsulfoxyde (DMSO) jusqu'à une concentration de 2,5 mM, puis stockés à une température de -20°C jusqu'à utilisation.

La lignée de cellules fibroblastiques pluripotentes C3H10T1/2 (ATCC CCL 226) a été cultivée dans les conditions recommandées par l'ATCC dans le milieu de culture DMEM supplémenté avec 10% de sérum de veau foetal à une température de 37°C sous atmosphère à 5 % de CO₂. 24 heures après ensemencement, la stimulation des cellules a été réalisée pendant 6 jours en présence des composés de formule générale (I) directement dilués dans le milieu de culture. L'activation par ces composés provoque la différenciation de la lignée cellulaire vers le lignage ostéoblastique et leur permet d'exprimer la phosphatase alcaline. Celle-ci est ensuite détectée par histochimie ou dosage enzymatique.

### Détection de la phosphatase alcaline par histochimie.

Pour cette expérience, les cellules sont cultivées en plaques de 6 puits contenant une lamelle en verre traitée 1H à la polyD-Lysine 0,05 mg/ml. Les cellules C3H10T1/2 sont ensemencées à une densité de 150 000 cellules par puits. Les composés ont été appliqués à une concentration de 10 µM. Le Kit de coloration SIGMA (85L-3R) a été utilisé en suivant le protocole décrit. Brièvement, après fixation des cellules 30 secondes par une solution de citrate/acétone (2 -3) puis rinçage à l'eau bi-distillée, la coloration est effectuée 30 min en présence d'une solution de Fast-Violet/Naphtol à l'abri de la lumière. Les lames sont ensuite rincées abondamment à l'eau bi distillée puis montée en milieu aqueux avant photographie sur un microscope DMRXA2 (Leica Microsystems). Les cellules exprimant la phosphatase alcaline sont colorées en rouge.

### Dosage enzymatique de l'alcaline phosphatase en plaques 96 puits.

Les cellules C3H10T1/2 ont été ensemencées sur des plaques de 96 puits à une densité de 5.10³ cellules par puits. Les composés ont été appliqués à des concentrations croissantes allant de 10 nM à 10 µM. Les plaques ont ensuite été incubées pendant 6 jours puis les essais ont été réalisés en quadruplicats selon les méthodes décrites précédemment (Chen et al. 2002; Frank-Kamenetsky et al. 2002).

Les cellules ont été lavées dans du tampon phosphate froid (PBS) puis lysées par sonication à 4°C dans 50 µl d'une solution contenant 0,9 % de NaCl et 0,2 % de Triton X-100. La mesure de l'activité phosphatase alcaline dans les lysats ainsi obtenus a été ensuite réalisée selon la méthode décrite par Pepinsky *et al.* (Pepinsky et al. 1998). Après addition de 100 µl de tampon réactionnel (200 mM Tris-HCl ; pH 10,5 ; 0,4 M de 2-amino-2-méthyl propanol et 8 mM de MgCl2) et de 50 µl de substrat (4 mM de p-Nitrophényl phosphate disodium), les lysats ont été incubés à 37°C pendant 60 min, puis la densité optique (DO) a été mesurée à une longueur d'onde de 415 nm. A titre comparatif, l'activité du composé SAG, activateur de l'ostéogenèse par action sur la voie hedgehog, a été testé dans les mêmes conditions. Le logiciel GraphPad Prism 4® a été utilisé pour tracer les courbes et déterminer les concentrations efficaces 50 (CE₅₀).

### II.2) Résultats

### II.2-1. Démonstration par histochimie de la différenciation des cellules souches mésenchymateuses par les composés de formule (I) vers le lignage ostéoblastique.

La stimulation de l'ostéogenèse dans les cellules pluripotentes C3H10T1/2 a été appréciée en observant l'induction de la phosphatase alcaline (PA). Après 6 jours de différenciation en présence de 10 µM de composés de formule (I), l'expression de la PA a été détectée par coloration histochimique. A titre d'exemple, une augmentation du nombre de cellules marquées est observée en présence des composés 1, 4, 6 et 8 avec des intensités plus ou moins fortes (composé 4 > composé 1 > composé 8 > composé 6). Aucune cellule traitée avec le solvant (DMSO) n'exprime la PA à un niveau détectable par cette méthode.

### II.2-2. Différenciation des cellules souches mésenchymateuses par les composés de formule (I) : dosage de l'activité de la phosphatase alcaline.

Les courbes doses réponses des composés de formule (I) ont été construites entre 10 nM et 10 µM et les affinités des composés vis-à-vis de l'activation de la différenciation des cellules C3H10T1/2 ont été déterminées. **Sur la** **Figure 3** sont présentées une courbe représentative du composé 1 réalisée en parallèle avec celle de l'agoniste du récepteur smoothened SAG. Ce dernier donne une stimulation maximum inférieure au composé 1 alors que leurs affinités sont proches.

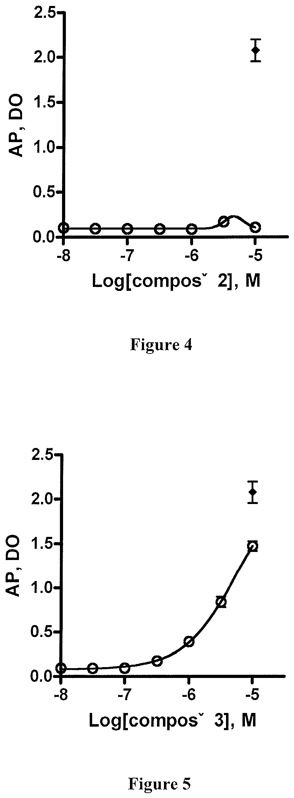

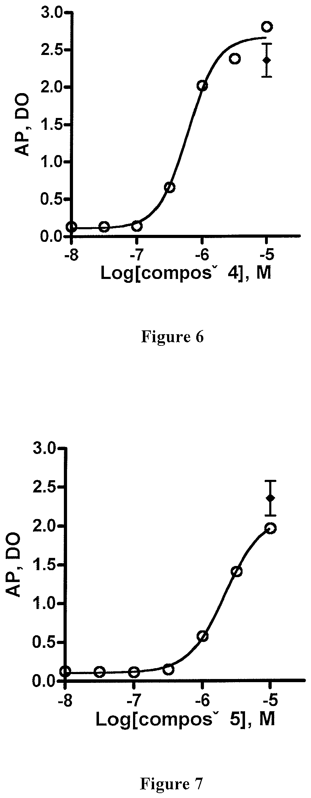

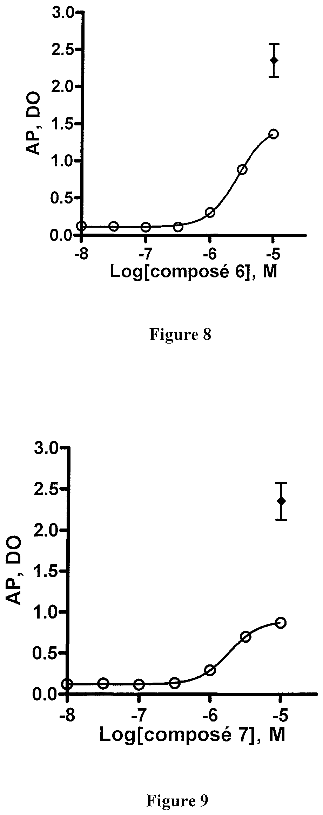

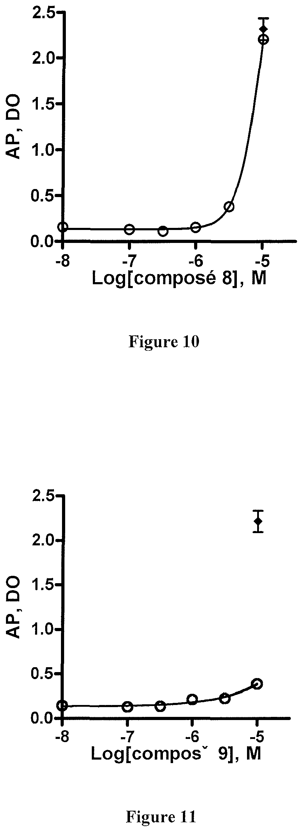

Les courbes obtenues pour les composés de 2 à 9 sont présentées sur les **Figures 4 à 11****.** Tous ces composés permettent la stimulation de la différenciation avec des différences caractéristiques pour chaque composé. Le maximum d'activité est observé à 10 µM pour la plupart d'entre eux.

Les résultats de l'exploitation de ces courbes sont reportés dans le Tableau I ci-après. Les affinités des composés sont de l'ordre du micromolaire. Le composé 2 est le moins actif avec un maximum de 4% du composé 1. A l'opposé, le composé 4 est celui qui présente la meilleure activité avec une affinité de 0,6 µm et un maximum de stimulation supérieur de 20% à celui du composé 1.

**Tableau I: Affinité et maximum d'activation des composés de formule (I) sur la différenciation des cellules multipotentes C3H10T1/2.**

| | Activité des composés de formule (I) sur la différenciation des cellules C3H10T1/2 mesurée par l'activité de la phosphatase alcaline. | |
|---|---|---|
| Composé | CE₅₀, µM | Maximum de stimulation, % de l'activation maximale du composé 1 |
| 1 | 1.3 | 100 |
| 2 | >3 | 4 |
| 3 | 3.6 | 66 |
| 4 | 0.6 | 124 |
| 5 | 1.5 | 91 |
| 6 | 3.1 | 62 |
| 7 | 2 | 47 |
| 8 | 5.8 | 94 |
| 9 | 6.5 | 33 |

La concentration efficace 50 (CE₅₀) des composés sur la différenciation est exprimé en µM. Le maximum de stimulation est exprimé en pourcentage de celui obtenu avec le composé 1 dans la même expérience. Les données correspondent à la moyenne de 2 à 5 expériences indépendantes.

### REFERENCES

Aghaloo, T. L., C. M. Amantea, et al. (2007). "Oxysterols enhance osteoblast differentiation in vitro and bone healing in vivo." J Orthop Res 25(11): 1488-97.
Amantea, C. M., W. K. Kim, et al. (2008). "Oxysterol-induced osteogenic differentiation of marrow stromal cells is regulated by Dkk-1 inhibitable and PI3-kinase mediated signaling." J Cell Biochem 105(2): 424-36.
Beloti, M. M., L. S. Bellesini, et al. (2005). "Purmorphamine enhances osteogenic activity of human osteoblasts derived from bone marrow mesenchymal cells." Cell Biol Int 29(7): 537-41.
Boonen, S., C. Rosen, et al. (2002). "Musculoskeletal effects of the recombinant human IGF-I/IGF binding protein-3 complex in osteoporotic patients with proximal femoral fracture: a double-blind, placebo-controlled pilot study." J Clin Endocrinol Metab 87(4): 1593-9.
Centrella, M., M. C. Horowitz, et al. (1994). "Transforming growth factor-beta gene family members and bone." Endocr Rev 15(1): 27-39.
Chen, J. K., J. Taipale, et al. (2002). "Small molecule modulation of Smoothened activity." Proc Natl Acad Sci U S A 99(22): 14071-6.
Corcoran, R. B. and M. P. Scott (2006). "Oxysterols stimulate Sonic hedgehog signal transduction and prolifération of medulloblastoma cells." Proc Natl Acad Sci U S A 103(22): 8408-13.
Dwyer, J. R., N. Sever, et al. (2007). "Oxysterols are novel activators of the hedgehog signaling pathway in pluripotent mesenchymal cells." J Biol Chem 282(12): 8959-68.
Frank-Kamenetsky, M., X. M. Zhang, et al. (2002). "Small-molecule modulators of Hedgehog signaling: identification and characterization of Smoothened agonists and antagonists." J Biol 1(2): 10.
Fromigue, O., D. Modrowski, et al. (2004). "Growth factors and bone formation in osteoporosis: roles for fibroblast growth factor and transforming growth factor beta." Curr Pharm Des 10(21): 2593-603.
Giustina, A., G. Mazziotti, et al. (2008). "Growth hormone, insulin-like growth factors, and the skeleton." Endocr Rev 29(5): 535-59.
Guan, C. C., M. Yan, et al. (2009). "Sonic hedgehog alleviates the inhibitory effects of high glucose on the osteoblastic differentiation of bone marrow stromal cells." Bone 45(6): 1146-52.
Hu, H., M. J. Hilton, et al. (2005). "Sequential roles of Hedgehog and Wnt signaling in osteoblast development." Development 132(1): 49-60.
Ingham, P. W. and A. P. McMahon (2001). "Hedgehog signaling in animal development: paradigms and principles." Genes Dev 15(23): 3059-87.
Johnson, E. E. and M. R. Urist (2000). "Human bone morphogenetic protein allografting for reconstruction of femoral nonunion." Clin Orthop Relat Res(371): 61-74.
Lum, L. and P. A. Beachy (2004). "The Hedgehog response network: sensors, switches, and routers." Science 304(5678): 1755-9.
Marti, E. and P. Bovolenta (2002). "Sonic hedgehog in CNS development: one signal, multiple outputs." Trends Neurosci 25(2): 89-96.
Mohammad, K. S., C. G. Chen, et al. (2009). "Pharmacologic inhibition of the TGF-beta type I receptor kinase has anabolic and anti-catabolic effects on bone." PLoS One 4(4): e5275.
Pepinsky, R. B., C. Zeng, et al. (1998). "Identification of a palmitic acid-modified form of human Sonic hedgehog." J Biol Chem 273(22): 14037-45.
Spinella-Jaegle, S., G. Rawadi, et al. (2001). "Sonic hedgehog increases the commitment of pluripotent mesenchymal cells into the osteoblastic lineage and abolishes adipocytic differentiation." J Cell Sci 114(Pt 11): 2085-94.
van der Horst, G., H. Farih-Sips, et al. (2003). "Hedgehog stimulates only osteoblastic differentiation of undifferentiated KS483 cells." Bone 33(6): 899-910.
Varjosalo, M. and J. Taipale (2008). "Hedgehog: functions and mechanisms." Genes Dev 22(18): 2454-72.
Wechsler-Reya, R. and M. P. Scott (2001). "The developmental biology of brain tumors." Annu Rev Neurosci 24: 385-428.
Wu, X., J. Walker, et al. (2004). "Purmorphamine induces osteogenesis by activation of the hedgehog signaling pathway." Chem Biol 11(9): 1229-38.
Yamaguchi, A., T. Komori, et al. (2000). "Regulation of osteoblast differentiation mediated by bone morphogenetic proteins, hedgehogs, and Cbfa1." Endocr Rev 21(4): 393-411.
Yu, P. B., C. C. Hong, et al. (2008). "Dorsomorphin inhibits BMP signals required for embryogenesis and iron metabolism." Nat Chem Biol 4(1): 33-41.

## Revendications

1. Composés de formule générale (I) : 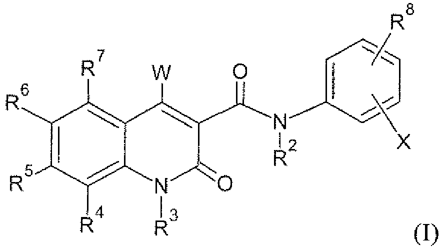 dans laquelle :
- X, positionné en ortho, méta ou para, représente -H, -OH, -NH₂, un atome d'halogène, un radical alkyle consistant en une chaine carbonée de 1 à 10 atomes de carbones, linéaire ou ramifiée, un radical alcoxy, un cycloalkyle de 3 à 8 atomes de carbone ou un groupement aryle ;
- R⁸, positionné en ortho, méta ou para, sur un carbone différent de celui qui porte le radical X, représente : -(C=O)-NH-R¹, -(C=O)-O-R¹ ou -NH-(C=O)-R¹ ;
- W représente -H, -OH, -NH₂ ou un atome d'halogène ;
- R¹, R² et R³ identiques ou différents et indépendamment les uns des autres, représentent :
- un atome d'hydrogène ; ou
- un groupe alkyle consistant en une chaine carbonée de 1 à 10 atomes de carbones, linéaire ou ramifiée, éventuellement insaturée par une ou plusieurs double ou triple liaisons, éventuellement substituée par un ou plusieurs hétéroatomes, par un ou plusieurs atomes d'halogènes ou par un ou plusieurs groupements aryles ou hétéroaryles ; ou
- un cycloalkyle de 3 à 8 atomes de carbones éventuellement substitué par un radical alkyle consistant en une chaine carbonée de 1 à 10 atomes de carbone, linéaire ou ramifiée ou par un radical alcoxy ;
- R⁴, R⁵, R⁶ et R⁷ identiques ou différents et indépendamment les uns des autres, sont choisis parmi -H, -Cl, -Br, -I, -CN, -NO₂, un radical alkyle consistant en une chaine carbonée de 1 à 10 atomes de carbone, linéaire ou ramifiée, un radical alcoxy ou un cycloalkyle de 3 à 8 atomes de carbone ;
étant entendu que R³ et R⁴ peuvent être fusionnés pour former, avec les atomes de carbone et d'azote adjacent du cycle quinoline qui les porte, un cycle à 5 ou 6 chaînons ;
pour leur utilisation pour le traitement des maladies causées par le dérèglement de la fonction ou de la différenciation des ostéoblastes et/ou par le déséquilibre fonctionnel entre les ostéoblastes et les ostéoclastes, ladite utilisation permet d'induire la différenciation cellulaire de cellules souches ou progénitrices.

2. Composés selon la revendication 1, pour le traitement de l'ostéoporose ; et des désordres tels que la fragilité du squelette et/ou raréfaction osseuse et/ou la fragilisation du tissu osseux résultant de l'ostéopénie ; l'ostéogenèse imparfaite ; l'hypercalcémie ; l'hyperparathyroïdie ; l'ostéomalacie, l'ostéonécrose ; la maladie osseuse de Paget (ostéite déformante) ; l'arthrite rhumatoïde ; l'arthrite inflammatoire ; l'ostéomyélite; la parodontite ; les métastases osseuses.

3. Implant de cellules autologues différenciées en ostéoblastes comprenant au moins un composé de formule générale (I) : 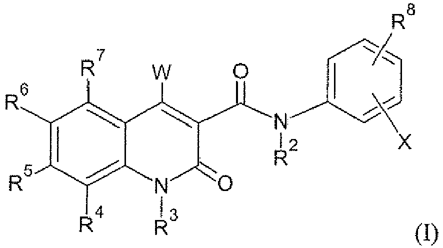 dans laquelle :
- X, positionné en ortho, méta ou para, représente -H, -OH, -NH₂, un atome d'halogène, un radical alkyle consistant en une chaine carbonée de 1 à 10 atomes de carbones, linéaire ou ramifiée, un radical alcoxy, un cycloalkyle de 3 à 8 atomes de carbone ou un groupement aryle ;
- R⁸, positionné en ortho, méta ou para, sur un carbone différent de celui qui porte le radical X, représente : -(C=O)-NH-R¹, -(C=O)-O-R¹ ou -NH-(C=O)-R¹ ;
- W représente -H, -OH, -NH₂ ou un atome d'halogène ;
- R¹, R² et R³ identiques ou différents et indépendamment les uns des autres, représentent :
- un atome d'hydrogène ; ou
- un groupe alkyle consistant en une chaine carbonée de 1 à 10 atomes de carbones, linéaire ou ramifiée, éventuellement insaturée par une ou plusieurs double ou triple liaisons, éventuellement substituée par un ou plusieurs hétéroatomes, par un ou plusieurs atomes d'halogènes ou par un ou plusieurs groupements aryles ou hétéroaryles ; ou
- un cycloalkyle de 3 à 8 atomes de carbones éventuellement substitué par un radical alkyle consistant en une chaine carbonée de 1 à 10 atomes de carbone, linéaire ou ramifiée ou par un radical alcoxy ;
- R⁴, R⁵, R⁶ et R⁷ identiques ou différents et indépendamment les uns des autres, sont choisis parmi -H, -Cl, -Br, -I, -CN, -NO₂, un radical alkyle consistant en une chaine carbonée de 1 à 10 atomes de carbone, linéaire ou ramifiée, un radical alcoxy ou un cycloalkyle de 3 à 8 atomes de carbone ;
étant entendu que R³ et R⁴ peuvent être fusionnés pour former, avec les atomes de carbone et d'azote adjacent du cycle quinoline qui les porte, un cycle à 5 ou 6 chaînons ;
pour son utilisation pour le traitement des pertes de tissu osseux consécutives à des blessures et/ou à des opérations chirurgicales par induction de la différenciation de cellules mésenchymateuses en ostéoblastes.

4. Composé selon la revendication 1 ou 2 pour son utilisation selon la revendication 1, **caractérisé en ce que** le radical R³ est un radical alkyle de 3 à 8 atomes de carbones ; le radical R² est un atome d'hydrogène ou un radical alkyle de 1 à 3 atomes de carbone ; que les radicaux R⁴, R⁵, R⁶ et R⁷ représentent un atome d'hydrogène ou un radical alkyle de 1 à 3 atomes de carbone.

5. Composé selon l'une quelconque des revendications 1, 2 ou 4 pour son utilisation selon la revendication 1, **caractérisés en ce qu'**il est choisi parmi :
- le propyl 4-(1-hexyl-4-hydroxy-2-oxo-1,2-dihydroquinoline-3-carboxamido)-benzoate **(composé 1) ;**
- l'éthyle 4-(1-benzyle-4-hydroxy-2-oxo-1,2-dihydroquinoline-3-carboxamido)-benzoate le **(composé 2) ;**
- l'éthyle 4-(1-hexyl-4-hydroxy-2-oxo-1,2-dihydroquinoline-3-carboxamido)-benzoate **(composé 3) ;**
- l'acide 4-(1-hexyl-4-hydroxy-2-oxo-1,2-dihydroquinoline-3-carboxamido)-benzoique **(composé 4) ;**
- le butyl 4-(1-hexyl-4-hydroxy-2-oxo-1,2-dihydroquinoline-3-carboxamido)-benzoate **(composé 5) ;**
- le tert-butyl 4-(1-hexyl-4-hydroxy-2-oxo-1,2-dihydroquinoline-3-carboxamido)-benzoate **(composé 6);**
- le benzyle 4-(1-hexyl-4-hydroxy-2-oxo-1,2-dihydroquinoline-3-carboxamido)-benzoate **(composé 7);**
- le N-(4-(butylcarbamoyl)phényl)-1-hexyl-4-hydroxy-2-oxo-1,2-dihydroquinoline-3-carboxamide **(composé 8) ;**
- l'isopropyl-4-(1-hexyl-4-hydroxy-2-oxo-1,2-dihydroquinoline-3-carboxamido)-benzoate **(composé 9).**

6. Implant selon la revendication 3 pour son utilisation selon la revendication 3, **caractérisé en ce que** ledit composé est tel que le radical R³ est un radical alkyle de 3 à 8 atomes de carbones ; le radical R² est un atome d'hydrogène ou un radical alkyle de 1 à 3 atomes de carbone ; que les radicaux R⁴, R⁵, R⁶ et R⁷ représentent un atome d'hydrogène ou un radical alkyle de 1 à 3 atomes de carbone.

7. Implant selon la revendication 3 ou 6 pour son utilisation selon la revendication 3, **caractérisé en ce que** ledit composé est choisi parmi :
- le propyl 4-(1-hexyl-4-hydroxy-2-oxo-1,2-dihydroquinoline-3-carboxamido)-benzoate **(composé 1) ;**
- l'éthyle 4-(1-benzyle-4-hydroxy-2-oxo-1,2-dihydroquinoline-3-carboxamido)-benzoate le **(composé 2) ;**
- l'éthyle 4-(1-hexyl-4-hydroxy-2-oxo-1,2-dihydroquinoline-3-carboxamido)-benzoate **(composé 3) ;**
- l'acide 4-(1-hexyl-4-hydroxy-2-oxo-1,2-dihydroquinoline-3-carboxamido)-benzoique **(composé 4) ;**
- le butyl 4-(1-hexyl-4-hydroxy-2-oxo-1,2-dihydroquinoline-3-carboxamido)-benzoate (**composé 5) ;**
- le tert-butyl 4-(1-hexyl-4-hydroxy-2-oxo-1,2-dihydroquinoline-3-carboxamido)-benzoate **(composé 6);**
- le benzyle 4-(1-hexyl-4-hydroxy-2-oxo-1,2-dihydroquinoline-3-carboxamido)-benzoate **(composé 7);**
- le N-(4-(butylcarbamoyl)phényl)-1-hexyl-4-hydroxy-2-oxo-1,2-dihydroquinoline-3-carboxamide **(composé 8) ;**
- l'isopropyl-4-(1-hexyl-4-hydroxy-2-oxo-1,2-dihydroquinoline-3-carboxamido)-benzoate **(composé 9).**

8. Procédé de préparation de cellules ostéoblastiques à partir de cellules souches adultes ou de cellules progénitrices comprenant une étape consistant à faire incuber pendant un temps suffisant lesdites cellules dans un milieu nutritif liquide permettant le développement desdites cellules, ledit milieu nutritif contenant en solution au moins un composé de formule générale (I) tel que défini dans les revendication 1, 2, 4 ou 5.

9. Composés de formule générale (la) : 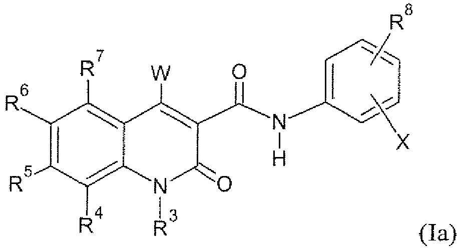 dans laquelle :
- X, positionné en ortho, méta ou para, représente -H, -OH, -NH₂, un atome d'halogène, un radical alkyle consistant en une chaine carbonée de 1 à 10 atomes de carbones, linéaire ou ramifiée, un radical alcoxy, un cycloalkyle de 3 à 8 atomes de carbone ou un groupement aryle ;
- R⁸, positionné en ortho, méta ou para, sur un carbone différent de celui qui porte le radical X, représente : -(C=O)-NH-R¹, -(C-O)-O-R¹ ou -NH-(C=O)-R¹ ;
- W représente -H, -OH, -NH₂ ou un atome d'halogène ;
- R¹ et R³, identiques ou différents, et indépendamment l'un de l'autre, représentent un groupe alkyle consistant en une chaine carbonée de 3 à 10 atomes de carbone, linéaire ou ramifiée, éventuellement insaturée par une ou plusieurs double ou triple liaisons, éventuellement substituée par un ou plusieurs hétéroatomes, par un ou plusieurs atomes d'halogènes ou par un ou plusieurs groupements aryles ou hétéroaryles ;
- R⁴, R⁵, R⁶ et R⁷ identiques ou différents et indépendamment les uns des autres, sont choisis parmi -H, -Cl, -Br, -I, -CN, -NO₂, un radical alkyle consistant en une chaine carbonée de 1 à 10 atomes de carbone, linéaire ou ramifiée, un radical alcoxy ou un cycloalkyle de 3 à 8 atomes de carbone ;
étant entendu que R³ et R⁴ peuvent être fusionnés pour former, avec les atomes de carbone et d'azote adjacent du cycle quinoline qui les porte, un cycle à 6 chaînons,
à l'exclusion du butyl 4-(1-propyl-4-hydroxy-2-oxo-1,2-dihydroquinoline-3-carboxamido)-benzoate.

10. Composés de formule générale (Ia) selon la revendication 9 en tant que médicament.

11. Composition pharmaceutique **caractérisée par le fait qu'**elle comprend, à titre de principe actif, au moins un composé de formule générale (Ia) selon la revendication 9, et au moins un excipient pharmaceutiquement acceptable.

## Patentansprüche

1. Verbindung mit der allgemeinen Formel (I): 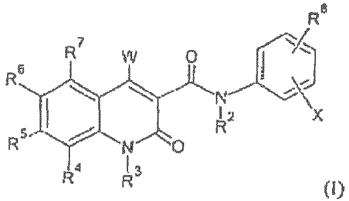 in der:
- X in der ortho-, meta- oder para-Stellung -H, -OH, -NH₂, ein Halogenatom, ein Alkylradikal, das aus einer linearen oder verzweigten Kohlenstoffkette mit 1 bis 10 Kohlenstoffatomen besteht, ein Alkoxyradikal, ein Cycloalkyl mit 3 bis 8 Kohlenstoffatomen oder eine Arylgruppe darstellt;
- R⁸ in der ortho-, meta- oder para-Stellung auf einem Kohlenstoff, der sich von demjenigen unterscheidet, der das Radikal X trägt, Folgendes darstellt: -(C=O)-NH-R¹, -(C=O)-O-R¹ oder -NH-(C=O)-R¹;
- W -H, -OH, -NH₂ oder ein Halogenatom darstellt;
- identische oder unterschiedliche R¹, R² und R³ und voneinander unabhängig Folgendes darstellen:
- ein Wasserstoffatom; oder
- eine Alkylgruppe, die aus einer linearen oder verzweigten Kohlenstoffkette mit 1 bis 10 Kohlenstoffatomen besteht, die gegebenenfalls durch eine oder mehrere Doppel- oder Dreifachbindungen ungesättigt ist, gegebenenfalls durch ein oder mehrere Heteroatome, durch ein oder mehrere Halogenatome oder durch eine oder mehrere Aryl- oder Heteroarylgruppen substituiert ist; oder
- ein Cycloalkyl mit 3 bis 8 Kohlenstoffatomen, das gegebenenfalls durch ein Alkylradikal, das aus einer linearen oder verzweigten Kohlenstoffkette mit 1 bis 10 Kohlenstoffatomen besteht, oder durch ein Alkoxyradikal substituiert ist;
- identische oder unterschiedliche R⁴, R⁵, R⁶ und R⁷ und voneinander unabhängig unter -H, -Cl, -Br, -I, -CN, -NO₂, einem Alkylradikal, das aus einer linearen oder verzweigten Kohlenstoffkette mit 1 bis 10 Kohlenstoffatomen besteht, einem Alkoxyradikal oder einem Cycloalkyl mit 3 bis 8 Kohlenstoffatomen ausgewählt sind;
wobei sich versteht, dass R³ und R⁴ fusioniert werden können, um mit den Kohlenstoff- und Stickstoffatomen dem Chinolinolring, der sie trägt, benachbart, einen Ring mit 5 oder 6 Gliedern zu bilden;
für ihre Verwendung zur Behandlung von Krankheiten, die durch Störung der Funktion oder der Differenzierung der Osteoblasten und/oder durch das funktionelle Ungleichgewicht zwischen den Osteoblasten und den Osteoklasten hervorgerufen werden, wobei die Verwendung das Bewirken der Zelldifferenzierung von Stamm- oder Progenitorzellen ermöglicht.

2. Verbindung nach Anspruch 1 zur Behandlung von Osteoporose; und Störungen wie Skelettschwäche und/oder Knochenauflockerung und/oder Schwächung des Knochengewebes infolge von Osteopenie; unvollkommene Osteogenese; Hyperkalzämie; Hyperparathyreoidismus; Osteomalazie; Osteonekrose; Paget-Knochenkrankheit (Ostitits deformans); rheumatoide Arthritis; entzündliche Arthritis; Osteomyelitis; Parodontitis; Knochenmetastasen.

3. Implantat von in Osteoblasten differenzierten autologen Zellen, das mindestens eine Verbindung mit der folgenden allgemeinen Formel (I) umfasst: 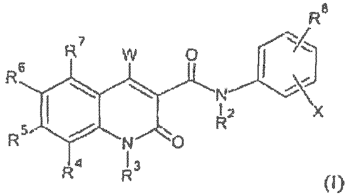 in der:
- X in der ortho-, meta- oder para-Stellung -H, -OH, -NH₂, ein Halogenatom, ein Alkylradikal, das aus einer linearen oder verzweigten Kohlenstoffkette mit 1 bis 10 Kohlenstoffatomen besteht, ein Alkoxyradikal, ein Cycloalkyl mit 3 bis 8 Kohlenstoffatomen oder eine Arylgruppe darstellt;
- R⁸ in der ortho-, meta- oder para-Stellung auf einem Kohlenstoff, der sich von demjenigen unterscheidet, der das Radikal X trägt, Folgendes darstellt: -(C=O)-NH-R¹, -(C=O)-O-R¹ oder -NH-(C=O)-R¹;
- W -H, -OH, -NH₂ oder ein Halogenatom darstellt;
- identische oder unterschiedliche R¹, R² und R³ und voneinander unabhängig Folgendes darstellen:
- ein Wasserstoffatom; oder
- eine Alkylgruppe, die aus einer linearen oder verzweigten Kohlenstoffkette mit 1 bis 10 Kohlenstoffatomen besteht, die gegebenenfalls durch eine oder mehrere Doppel- oder Dreifachbindungen ungesättigt ist, gegebenenfalls durch ein oder mehrere Heteroatome, durch ein oder mehrere Halogenatome oder durch eine oder mehrere Aryl- oder Heteroarylgruppen substituiert ist; oder
- ein Cycloalkyl mit 3 bis 8 Kohlenstoffatomen, gegebenenfalls durch ein Alkylradikal, das aus einer linearen oder verzweigten Kohlenstoffkette mit 1 bis 10 Kohlenstoffatomen besteht, oder durch ein Alkoxyradikal substituiert ist;
- identische oder unterschiedliche R⁴, R⁵, R⁶ und R⁷ und voneinander unabhängig unter -H, -Cl, -Br, -I, -CN, -NO₂, einem Alkylradikal, das aus einer linearen oder verzweigten Kohlenstoffkette mit 1 bis 10 Kohlenstoffatomen besteht, einem Alkoxyradikal oder einem Cycloalkyl mit 3 bis 8 Kohlenstoffatomen ausgewählt sind;
wobei sich versteht, dass R³ und R⁴ fusioniert werden können, um mit den Kohlenstoff- und Stickstoffatomen dem Chinolinolring, der sie trägt, benachbart einen Ring mit 5 oder 6 Gliedern zu bilden;
für seine Verwendung zur Behandlung von Verlusten an Knochengewebe nach Verletzungen und/oder chirurgischen Eingriffen durch Induktion der Differenzierung von mesenchymalen Zellen zu Osteoblasten.

4. Verbindung nach Anspruch 1 oder 2 für ihre Verwendung nach Anspruch 1, **dadurch gekennzeichnet, dass** das Radikal R³ ein Alkylradikal mit 3 bis 8 Kohlenstoffatomen ist; das Radikal R² ein Wasserstoffatom oder ein Alkylradikal mit 1 bis 3 Kohlenstoffatomen ist; dass die Radikale R⁴, R⁴, R⁶ und R⁷ ein Wasserstoffatom oder ein Alkylradikal mit 1 bis 3 Kohlenstoffatomen darstellen.

5. Verbindung nach einem der Ansprüche 1, 2 oder 4 für ihre Verwendung nach Anspruch 1, **dadurch gekennzeichnet, dass** sie unter Folgendem ausgewählt ist:
- Propyl-4-(1-hexyl-4-hydroxy-2-oxo-1,2-dihydrochinolin-3-carboxamido)-benzoat (Verbindung 1);
- Ethyl-4-(1-benzyl-4-hydroxy-2-oxo-1,2- dihydrochinolin-3-carboxamido)-benzoat (Verbindung 2);
- Ethyl-4-(1-hexyl-4-hydroxy-2-oxo-1,2-dihydrochinolin-3-carboxamido)-benzoat (Verbindung 3);
- 4-(1-hexyl-4-hydroxy-2-oxo-1,2,-dihydrochinolin-3-carboxamido)-benzolsäure (Verbindung 4);
- Butyl-4-(1-hexyl-4-hydroxy-2-oxo-1,2-dihydrochinolin-3-carboxamido)-benzoat (Verbindung 5);
- tert-Butyl-4-(1-hexyl-4-hydroxy-2-oxo-1,2-dihydrochinolin-3-carboxamido)-benzoat (Verbindung 6);
- Benzyl-4-(1-hexyl-4-hydroxy-2-oxo-1,2-dihydrochinolin-3-carboxamido)-benzoat (Verbindung (7);
- N-(4-(Butylcarbamoyl)phenyl)-1-hexyl-4-hydroxy-2-oxo-1,2-dihydrochinolin-3-carboxamid (Verbindung 8);
- Isopropyl-4-(1-hexyl-4-hydroxy-2-oxo-1,2-dihydrochinolin-3-carboxamido)-benzoat (Verbindung 9).

6. Implantat nach Anspruch 3 für seine Verwendung nach Anspruch 3, **dadurch gekennzeichnet, dass** die Verbindung derart ist, dass das Radikal R³ ein Alkylradikal mit 3 bis 6 Kohlenstoffatomen ist; das Radikal R² ein Wasserstoffatom oder ein Alkylradikal mit 1 bis 3 Kohlenstoffatomen ist; dass die Radikale R⁴, R⁵, R⁶ und R⁷ ein Wasserstoffatom oder ein Alkylradikal mit 1 bis 3 Kohlenstoffatomen darstellen.

7. Implantat nach Anspruch 3 oder 6 für seine Verwendung nach Anspruch 3, **dadurch gekennzeichnet, dass** die Verbindung unter Folgendem ausgewählt ist:
- Propyl-4-(1-hexyl-4-hydroxy-2-oxo-1,2-dihydrochinolin-3-carboxamido)-benzoat (Verbindung 1);
- Ethyl-4-(1-benzyl-4-hydroxy-2-oxo-1,2- dihydrochinolin-3-carboxamido)-benzoat (Verbindung 2);
- Ethyl-4-(1-hexyl-4-hydroxy-2-oxo-1,2-dihydrochinolin-3-carboxamido)-benzoat (Verbindung 3);
- 4-(1-hexyl-4-hydroxy-2-oxo-1,2,-dihydrochinolin-3-carboxamido)-benzolsäure (Verbindung 4);
- Butyl-4-(1-hexyl-4-hydroxy-2-oxo-1,2-dihydrochinolin-3-carboxamido)-benzoat (Verbindung 5);
- tert-Butyl-4-(1-hexyl-4-hydroxy-2-oxo-1,2-dihydrochinolin-3-carboxamido)-benzoat (Verbindung 6);
- Benzyl-4-(1-hexyl-4-hydroxy-2-oxo-1,2-dihydrochinolin-3-carboxamido)-benzoat (Verbindung (7);
- N-(4-(Butylcarbamoyl)phenyl)-1-hexyl-4-hydroxy-2-oxo-1,2-dihydrochinolin-3-carboxamid (Verbindung 8);
- Isopropyl-4-(1-hexyl-4-hydroxy-2-oxo-1,2-dihydrochinolin-3-carboxamido)-benzoat (Verbindung 9).

8. Verfahren zur Zubereitung von osteoblastischen Zellen aus adulten Stammzellen oder Progenitorzellen, das einen Schritt umfasst, der darin besteht, die Zellen während eines ausreichenden Zeitraums in einem flüssigen Nährmedium inkubieren zu lassen, das die Entwicklung der Zellen ermöglicht, wobei das Nährmedium mindestens eine Verbindung mit der allgemeinen Formel (I) nach Anspruch 1, 2, 4 oder 5 in Lösung umfasst.

9. Verbindung mit der allgemeinen Formel (Ia): 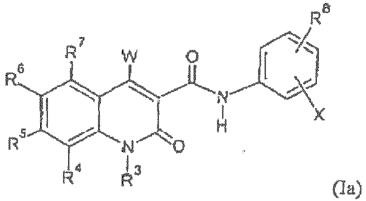 in der:
- X in der ortho-, meta- oder para-Stellung -H, -OH,
- NH₂, ein Halogenatom, ein Alkylradikal, das aus einer linearen oder verzweigten Kohlenstoffkette mit 1 bis 10 Kohlenstoffatomen besteht, ein Alkoxyradikal, ein Cycloalkyl mit 3 bis 8 Kohlenstoffatomen oder eine Arylgruppe darstellt;
- R⁸ in der ortho-, meta- oder para-Stellung auf einem Kohlenstoff, der sich von demjenigen unterscheidet, der das Radikal X trägt, Folgendes darstellt: -(C=O)-NH-R¹, -(C=O)-O-R¹ oder -NH-(C=O)-R¹;
- W -H, -OH, -NH₂ oder ein Halogenatom darstellt;
- identische oder unterschiedliche R¹ und R³ und voneinander unabhängig eine Alkylgruppe darstellen, die aus einer linearen oder verzweigten Kohlenstoffkette mit 3 bis 10 Kohlenstoffatomen besteht, die gegebenenfalls durch eine oder mehrere Doppel- oder Dreifachbindungen ungesättigt ist, gegebenenfalls durch ein oder mehrere Heteroatome, durch ein oder mehrere Halogenatome oder durch eine oder mehrere Aryl-oder Heteroarylgruppen substituiert ist;
- identische oder verschiedene R⁴, R⁵, R⁶ und R⁷ und voneinander unabhängig unter -H, -Cl, -Br, -I, -CN, NO₂, einem Alkylradikal, das aus einer linearen oder verzweigten Kohlenstoffkette mit 1 bis 10 Kohlenstoffatomen besteht, einem Alkoxyradikal oder einem Cycloalkyl mit 3 bis 8 Kohlenstoffatomen ausgewählt werden;
wobei es sich versteht, dass R³ und R⁴ fusioniert werden können, um mit den Kohlenstoff- und Stickstoffatomen dem Chinolinring, der sie trägt, benachbart unter Ausschluss des Butyl-4-(1-Propyl-4-hydroxy-2-oxo-1,2-dihydrochinolin-3-carboxamido)-benzoats einen Ring mit 6 Gliedern zu bilden.

10. Verbindungen mit der allgemeinen Formel (Ia) nach Anspruch 9 als Medikament.

11. Pharmazeutische Verbindung, **dadurch gekennzeichnet, dass** sie als Wirkstoff mindestens eine Verbindung mit der allgemeinen Formel (Ia) nach Anspruch 9 und mindestens einen pharmazeutisch akzeptablen Träger umfasst.

## Claims

1. Compounds of general formula (I) 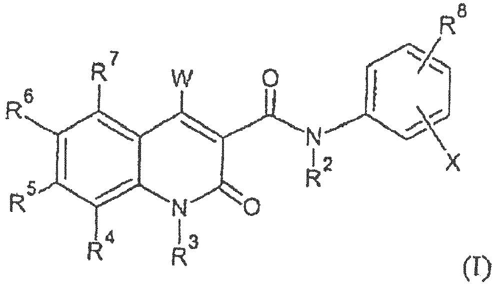 where:
- X, at ortho, meta or para position, represents -H, -OH, -NH₂, a halogen atom, an alkyl radical formed of a carbon chain having 1 to 10 carbon atoms, straight-chain or branched, an alkoxy radical, a cycloalkyl having 3 to 8 carbon atoms or an aryl group;
- R⁸, at ortho, meta or para position, on a carbon differing from that carrying the radical X, represents: -(C=O)-NH-R¹, -(C=O)-O-R¹ or -NH-(C=O)-R¹;
- W is -H, -OH, -NH₂ or a halogen atom;
- R¹, R² and R³, the same or different, are each independently:
∘ a hydrogen atom; or
∘ an alkyl group formed of a carbon chain having 1 to 10 carbon atoms, straight-chain or branched, optionally unsaturated with one or more double or triple bonds, optionally substituted by one of more heteroatoms, by one or more halogen atoms or by one or more aryl or heteroaryl groups; or
∘ a cycloalkyl having 3 to 8 carbon atoms optionally substituted by an alkyl radical formed of a carbon chain having 1 to 10 carbon atoms, straight-chain or branched, or by an alkoxy radical;
- R⁴, R⁵, R⁶ and R⁷, the same or different, are each independently selected from among-H, -Cl, -Br, -I, -CN, -NO₂, an alkyl radical formed of a carbon chain having 1 to 10 carbon atoms, straight-chain or branched, an alkoxy radical or cycloalkyl having 3 to 8 carbon atoms;
on the understanding that R³ and R⁴ may be fused so that, together with the carbon and nitrogen atoms adjacent to their carrier quinoline ring, they form a 5 or 6-membered ring;
for use thereof to treat diseases caused by deranged function or differentiation of osteoblasts and/or by functional imbalance between osteoblasts and osteoclasts, said use allowing the inducing of cell differentiation of stem or progenitor cells.

2. The compounds according to claim 1, for the treatment of osteoporosis; and of disorders such as skeletal fragility and/or bone rarefaction and/or weakened bone tissue resulting from osteopenia; osteogenesis imperfecta; hypercalcaemia; hyperparathyroidism; osteomalacia; osteonecrosis; Paget's disease of bone (osteitis deformans); rheumatoid arthritis; inflammatory arthritis; osteomyelitis, periodontitis; bone metastases.

3. Implant of autologous cells differentiated into osteoblasts, comprising at least one compound of general formula (I): 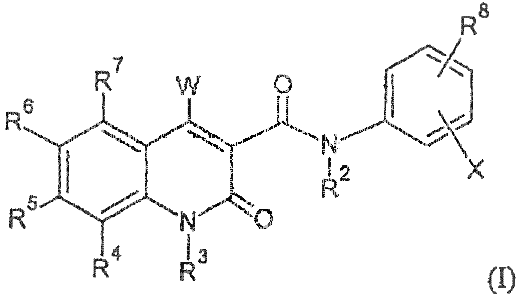 where:
- X, at ortho, meta or para position is -H, OH, -NH2, a halogen atom, an alkyl radical formed of a carbon chain having 1 to 10 carbon atoms, straight-chain or branched, an alkoxy radical, a cycloalkyl having 3 to 8 carbon atoms or an aryl group;
- R⁸ at ortho, meta or para position, on a carbon differing from that carrying the radical X, represents: -(C=O)-NH-R¹, -(C=O)-O-R¹ or -NH-(C=O)-R¹;
- W is -H, -OH. -NH₂ or a halogen atom;
- R¹, R² and R³, the same or different, are each independently:
o a hydrogen atom; or
o an alkyl group formed of a carbon chain having 1 to 10 carbon atoms, straight-chain or branched, optionally unsaturated with one or more double or triple bonds, optionally substituted by one or more heteroatoms, by one or more halogen atoms or by one or more aryl or heteroaryl groups; or
o a cycloalkyl having 3 to 8 carbon atoms optionally substituted by an alkyl radical formed of a carbon chain having 1 to 10 carbon atoms, straight-chain or branched, or by an alkoxy radical;
- R⁴, R⁵, R⁶ and R⁷, the same or different, are each independently selected from among -H, -Cl, -Br, -I, -CN, -NO₂, an alkyl radical formed of a carbon chain having 1 to 10 carbon atoms, straight-chain or branched, an alkoxy radical or a cycloalkyl having 3 to 8 carbon atoms;
on the understanding that R³ and R⁴ can be fused so that, together with the carbon and nitrogen atoms adjacent to their carrier quinoline ring, they form a 5- or 6-membered ring;
for use thereof to treat bone tissue loss subsequent to injury and/or surgery, by inducing differentiation of mesenchyme cells into osteoblasts.

4. The compound according to claim 1 or 2 for use thereof according to claim 1, **characterized in that** the radical R³ is an alkyl radical having 3 to 8 carbon atoms; radical R² is a hydrogen atom or alkyl radical having 1 to 3 carbon atoms; the radicals R⁴, R⁵, R⁶ and R⁷ are a hydrogen atom or an alkyl radical having 1 to 3 carbon atoms.

5. The compound according to any of claims 1, 2 or 4, for use thereof according to claim 1, **characterized in that** it is selected from among:
- propyl 4-(1-hexyl-4-hydroxy-2-oxo-1,2-dihydroquinoline-3-carboxamido)-benzoate **(compound 1);**
- ethyl 4-(1-benzyl-4-hydroxy-2-oxo-1,2-dihydroquinoline-3-carboxamido)-benzoate **(compound 2);**
- ethyl 4-(1-hexyl-4-hydroxy-2-0x0-1,2-dihydroquinoline-3-carboxamido)-benzoate 8 **(compound 3);**
- 4-(1-hexyl-4-hydroxy-2-oxo-1,2-dihydroquinoline-3-carboxamido) benzoic acid **(compound 4);**
- butyl 4-(1-hexyl-4-hydroxy-2-oxo-1,2-dihydroquinoline-3-carboxamido)-benzoate **(compound 5);**
- tert-butyl 4-(1-hexyl-4-hydroxy-2-oxo-1,2-dihydroquinoline-3-carboxamido)-benzoate **(compound 6);**
- benzyl 4-(1-hexyl-4-hydroxy-2-oxo-1,2-dihydroquinoline-3-carboxamido)-benzoate **(compound 7);**
- N-(4-(butylcarbamoyl)phenyl)-1-hexyl-4-hydroxy-2-oxo-1,2-dihydroquinoline-3-carboxamide **(compound 8);**
- isopropyl 4-(1-hexyl-4-hydroxy-2-oxo-1,2-dihydroquinoline-3-carboxamido)-benzoate **(compound 9);**

6. Implant according to claim 3 for use thereof according to claim 3, **characterized in that** said compound is such that radical R³ is an alkyl radical having 3 to 8 carbon atoms; radical R² is a hydrogen atom or alkyl radical having 1 to 3 carbon atoms; radicals R⁴, R⁵, R⁶ and R⁷ are a hydrogen atom or an alkyl radical having 1 to 3 carbon atoms.

7. implant according to claim 3 or 6 for use thereof according to claim 3, **characterized in that** said compound is selected from among:
- propyl 4-(1-hexyl-4-hydroxy-2-oxo-1,2-dihydroquinoline-3-carboxamido)-benzoate **(compound 1);**
- ethyl 4-(1-benzyl-4-hydroxy-2-oxo-1,2-dihydroquinoline-3-carboxamido)-benzoate **(compound 2);**
- ethyl 4-(1-hexyl-4-hydroxy-2-oxo-1,2-dihydroquinoline-3-carboxamido)-benzoate **(compound 3);**
- 4-(1-hexyl-4-hydroxy-2-oxo-1,2-dihydroquinoline-3-carboxamido) benzoic acid **(compound 4);**
- butyl 4-(1-hexyl-4-hydroxy-2-oxo-1,2-dihydroquinoline-3-carboxamido)-benzoate **(compound 5);**
- tert-butyl 4-(1-hexyl-4-hydroxy-2-oxo-1,2-dihydroquinoline-3-carboxamido)-benzoate **(compound 6);**
- benzyl 4-(1-hexyl-4-hydroxy-2-oxo-1,2-dihydroquinoline-3-carboxamido)-benzoate **(compound 7);**
- N-(4-(butylcarbamoyl)phenyl-1-hexyl-4-hydroxy-2-oxo-1,2-dihydroquinoline-3-carboxamide **(compound 8);**
- isopropyl 4-(1-hexyl-4-hydroxy-2-oxo-1,2-dihydroquinoline-3-carboxamido)-benzoate **(compound 9);**

8. Method for preparing osteoblast cells from adult stem cells or progenitor cells comprising a step consisting of incubating said cells for sufficient time in a liquid nutrient medium allowing the development of said cells, said nutrient medium containing in solution at least one compound of general formula (I) such as defined in claims 1,2, 4 or 5.

9. Compounds of general formula (Ia): 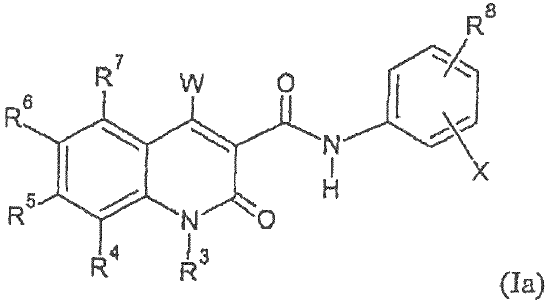 where:
- X, at ortho, meta or para position is -H, -OH, -NH₂, a halogen atom, an alkyl radical formed of a carbon chain having 1 to 10 carbon atoms, straight-chain or branched, an alkoxy radical, a cycloalkyl having 3 to 8 carbon atoms or an aryl group;
- R⁸, at ortho, meta or para position, on a carbon differing from that carrying the radical X, represents: -(C=O)-NH-R¹, -(C=O)-O-R¹ or -NH-(C=O)-R¹;
- W is -H, -OH, -NH₂ or a halogen atom;
- R¹ and R³, the same or different, are each independently an alkyl group formed of a carbon chain having 3 to 10 carbon atoms, straight-chain or branched, optionally unsaturated with one or more double or triple bonds, optionally substituted by one or more heteroatoms, by one or more halogen atoms, or by one or more aryl or heteroaryl groups;
- R⁴, R⁵, R⁶ and R⁷, the same or different, are each independently selected from among -H, -Cl, -Br, -I, -CN, -NO₂, an alkyl radical formed of a carbon chain having 1 to 10 carbon atoms, straight-chain or branched, an alkoxy radical or cycloalkyl having 3 to 8 carbon atoms;
on the understanding that R³ and R⁴ may be fused so that, together with the carbon and nitrogen atoms adjacent to their carrier quinoline ring, they form a 6-membered ring,
with the exclusion of butyl 4-(1-propyl-4-hydroxy-2-oxo-1,2-dihydroquinoline-3-carboxamido)-benzoate.

10. Compounds of general formula (Ia) according to claim 9 as medicinal product.

11. Pharmaceutical composition **characterized by** the fact that, as active ingredient, it comprises at least one compound of general formula (Ia) according to claim 9, and at least one pharmaceutically acceptable excipient.
